# EUROPÄISCHE PATENTSCHRIFT

(11) **EP 0 869 915 B1**
(45) Veröffentlichungstag und Bekanntmachung des Hinweises auf die Patenterteilung: **07.04.2004**
(21) Anmeldenummer: 96946244.9
(22) Anmeldetag: 19.12.1996
(51) Int. Cl.: C01F 5/14, C01F 5/08, C07C 29/70

(54) **VERFAHREN ZUR HERSTELLUNG VON HOCHREINEM MAGNESIUMHYDROXID UND MAGNESIUMOXID AUS MAGNESIUMALKOXIDEN**
PROCESS FOR THE PREPARATION OF HIGH-PURITY MAGNESIUM HYDROXIDE AND MAGNESIUM OXIDE FROM MAGNESIUM ALKOXIDES
PROCEDE DE PREPARATION D'HYDROXYDE DE MAGNESIUM TRES PUR ET D'OXYDE DE MAGNESIUM A PARTIR D'ALCOXYDES DE MAGNESIUM

(30) Priorität: 27.12.1995 DE 19548863
(43) Veröffentlichungstag der Anmeldung: 14.10.1998
(73) Patentinhaber: SASOL Germany GmbH, 22297 Hamburg (DE)
(72) Erfinder: BRASCH, Andrea, D-25704 Meldorf (DE); DIBLITZ, Klaus, D-22869 Schenefeld (DE); DÖLLING, Kai, D-25421 Pinneberg (DE); FELDBAUM, Tilo, D-20357 Hamburg (DE); NOWECK, Klaus, D-25541 Brunsbüttel (DE); SCHIEFLER, Jan, D-22605 Hamburg (DE)
(74) Vertreter: Schupfner, Gerhard D., Dr. Dipl.-Chem.
(86) Internationale Anmeldenummer: PCT/DE1996/002483
(87) Internationale Veröffentlichungsnummer: WO 1997/024304

(56) Entgegenhaltungen:
- GB-A- 667 708
- US-A- 3 657 361
- RESEARCH DISCLOSURE, Nr. 157, Mai 1977, Seite 19 XP002033210 "improved alkoxide productions"
- DATABASE WPI Section Ch, Week 8401 Derwent Publications Ltd., London, GB; Class E33, AN 84-005119 XP002033294 & SU 1 002 243 A (STEBNIK POTASSIUM WKS) , 7.März 1983

## Beschreibung

Die Erfindung betrifft ein Verfahren zur Herstellung von Magnesiumhydroxid hoher Reinheit sowie daraus durch Kalzinieren gewinnbares Magnesiumoxid.

Natürliche Vorkommen von Magnesiumhydroxid [CAS-Nr. 1309-48-4] sind sehr selten und werden kaum abgebaut. Magnesiumhydroxid wird heutzutage durch Ausfällung aus Seewasser (W. C. Gilpin, N. Heasman, Chem. Ind. (London), 1977, 567-572 sowie J. C. Drum, S. Tangney, Trans. J. Br. Ceramic Soc. 77 (1978) No. 4, 10-14) und Ausfällung aus Lösungen von Magnesiumsalzen mittels Calciumhydroxid gewonnen.

Die oben genannten Herstellungsverfahren haben den Nachteil, daß das derart gewonnene Magnesiumhydroxid für viele katalytische Anwendungen oder zur Herstellung von speziellen Keramiken nur unzureichend geeignet ist. Dies liegt besonders an den nicht zu vermeidenden Verunreinigungen durch andere Metalle, was insbesondere katalytische Anwendungen ausschließt.

Ein kontinuierliches Verfahren zur Herstellung von hochreinen Aluminiumalkoholaten ist in der DE 3 244 972-C1 beschrieben. Dabei wird Aluminiummetall mit Alkohol zum Aluminiumalkoholat umgesetzt. Dieses kann von im Aluminiummetall vorliegenden weiteren Metallen durch Filtration und/oder Destillation befreit werden, da diese Metalle sich nicht oder nur erheblich langsamer zu Metallalkoholaten umsetzen. Dieses Verfahren läßt sich jedoch bisher nicht auf Magnesium anwenden, da die bisher bekannten Magnesiümalkoholate nicht flüssig und daher nicht filtrierbar sind. Sie sind ebenfalls nicht unzersetzt schmelzbar und daher auch nicht destillierbar.

Verfahren zur Herstellung reiner Magnesiumhydroxide sind bekannt. GB-A-667 708 schlägt zum Beispiel ein dreistufiges Verfahren vor, in dem zunächst mit einem wasserlöslichen C1-oder C2- Alkohol zum Magnesiumalkoholat umgesetzt wird. Dieser wird in einer zweite Umsetzung durch einen längerkettigen Alkohol ausgetauscht, wobei der C1- oder C2- Alkohol durch Destillation entfernt wird. Erst dieses Magnesiumalkoholat wird in Schritt 3 hydrolysiert.

Die Herstellung von Magnesiumalkoxyethern aus Hydroxyethern ist an sich bekannt, siehe z.B. die US 3,657,361. Ein Hinweis, daß hochreine Magnesiumhydroxide mit besonderen Produkteigenschaften zugänglich sind, wenn Magnesiumalkoxyether hydrolysiert werden, ist daraus jedoch nicht entnehmbar.

Die EP 0 244 917 beschreibt ein Verfahren zur Herstellung von löslichen Metallalkoxiden aus Alkoxyalkoholen in organischen Lösungsmitteln, gibt jedoch keinen Hinweis auf eine Verfahren zur Herstellung von hochreinen kristallinen Magnesiumhydroxiden. mit enger Porenverteilung und einheitlicher Kristallinität.

Der Erfindung lag die Aufgabe zugrunde, ein Verfahren zur Herstellung von Magnesiumhydroxid zu entwickeln, das die nachfolgenden Merkmale aufweist:
- Das erfindungsgemäß hergestellte Magnesiumhydroxid soll von hoher Reinheit, enger Porenverteilung und einheitlicher Kristallinität sein.
- Die Ausgangsstoffe sollen preiswert und leicht verfügbar sein.
- Der Herstellungsprozeß soll kontinuierlich und diskontinuierlich durchführbar sein.

Überraschenderweise wurde nun gefunden, daß mit dem nachstehend beschriebenen Verfahren diese Aufgaben ohne weiteres gelöst werden können.

Die vorliegende Erfindung besteht aus einem Verfahren zur kontinuierlichen oder diskontinuierlichen Herstellung von Magnesiumhydroxid und/oder Magnesiumoxid in hochreiner Form, indem man Magnesiummetall und reaktive Magnesiumverbindungen mit Hydroxy-Verbindungen des Typs R¹-A-R-OH umsetzt und hydrolysiert und ggf. zur Herstellung des Magnesiumoxids anschließend kalziniert. Ggf. erfolgt die Umsetzung zusammen mit bis zu 50 Gew.% Alkoholen des Typs R²-OH .

Die erfindungsgemäß zu verwendenden Hydroxy-Verbindungen weisen die allgemeine Formel R¹-A-R-OH auf. A steht für ein Element aus der 6. Hauptgruppe des Periodensystems (beginnend mit Sauerstoff) oder der 5. Hauptgruppe des Periodensystems (beginnend mit Stickstoff). Steht A für die Stickstoff-Gruppe, so kann A zur Absättigung seiner Valenzen weitere Substituenten tragen, bevorzugt insgesamt 3 Substituenten. Diese können Wasserstoff oder ein weiterer ggf. von den anderen verschiedener Kohlenwasserstoffrest R¹ sein. Bevorzugt für die Heteroatome A sind Sauerstoff und Stickstoff, besonders bevorzugt für A ist Sauerstoff.

R, R¹ und R² stehen für einen verzweigten oder unverzweigten, cyclischen oder acyclischen, gesättigten, ungesättigten oder aromatischen Kohlenwasserstoffrest mit 1 - 10 Kohlenstoffatomen, wobei R, R¹ und R² voneinander verschieden sein können und R zweifach substituiert (divalent) ist.

Bevorzugt ist R¹ ein gesättigter Alkylrest mit 1 - 10 Kohlenstoffatomen, insbesondere ein Alkylrest mit 1 - 5 Kohlenstoffatomen, wobei dieser besonders bevorzugt unverzweigt ist. Bevorzugt ist R² ein verzweigter oder unverzweigter, cyclischer oder acyclischer, gesättigter Kohlenwasserstoffrest mit 4 - 8 Kohlenstoffatomen wobei dieser besonders bevorzugt unverzweigt, acyklisch und gesättigt ist. Bevorzugt sind für R verzweigte oder unverzweigte acyclische Alkylidenreste mit 1 - 5 Kohlenstoffatomen, insbesondere unverzweigte Kohlenwasserstoffe mit 1 bis 3 Kohlenstoffatomen.

Der Erfindung zugrunde liegt der Verfahrensschritt zur kontinuierlichen oder diskontinuierlichen Herstellung von flüssigen Magnesiumalkoxiden durch Umsetzung von geeigneten Hydroxy-Verbindungen mit gegenüber diesen reaktiven Magnesiumverbindungen und/oder Magnesiummetall. Eine geeignete reaktive Magnesiumverbindung ist z.B. Magnesiumhydrid. Der Einsatz von Magnesiummetall ist besonders bevorzugt. Überraschend hat sich gezeigt, daß organische Verbindungen des Typs R¹-A-R-OH nach Umsetzung mit Magnesium bzw. reaktiven Magnesiumverbindungen zu schon bei Raumtemperatur flüssigen Magnesiumalkoxiden führen.

Die Erfindung beinhaltet weiter die kontinuierliche oder diskontinuierliche Hydrolyse dieser flüssigen Magnesiumalkoxide, insbesondere Magnesiumalkoxyether und Magnesiumalkoxyamine, nach Abtrennung schwerer löslicher Verunreinigungen z.B. durch Filtration, Zentrifugieren oder Dekantieren zur Darstellung des hochreinen Magnesiumhydroxids, wobei eine gute Phasentrennung zwischen dem Wasser-Magnesiumhydroxid-Gemisch und den Alkoholkomponeneten nach der Hydrolyse durch den Zusatz fremdmetallionenfreier zum Aussalzen geeigneter Verbindungen wie insbesondere von 0,1 bis 10 Gew.% Ammoniumhydrogencarbonat zum Hydrolysewasser erreicht wird.

Die oben genannten Hydroxy-Verbindungen des Typs R¹-A-R-OH können alleine (eine Verbindung) oder als Gemisch (mehrere Verbindungen des Typs R¹-A-R-OH) eingesetzt werden. Insbesondere wenn bei der erfindungsgemäßen Umsetzung höherviskose Verbindungen entstehen, wird das Absetzen der Verunreinigungen in Form von schwerlöslichen Feststoffen behindert. Dann kann, unter anderem zur Erniedrigung der Viskosität und damit auch zur leichteren Filtrierbarkeit, ein 1- bis 5-facher Überschuß der Hydroxy-Verbindungen gewählt werden, wobei man dabei auch Alkohole vom Typ R²-OH einsetzen bzw. deren Anteil erhöhen kann. Besonders bevorzugt geschieht die Einstellung der Viskosität der Flüssigkeit/Lösung durch die nachträgliche Zugabe von Alkoholen des Typs R²-OH.

Ein Teil der bei der Umsetzung eingesetzten Hydroxy-Verbindungen vom Typ R¹-A-R-OH kann vor oder nach der Umsetzung durch andere Alkohole, nämlich solche vom Typ R²-OH ersetzt werden. Der Anteil der Alkohole vom Typ R²-OH darf jedoch insgesamt 50 Gew.% der Gesamtheit aller einzusetzenden Edukte bzw. Lösungsmittel nicht überschreiten.

Die Umsetzung der Hydroxy-Verbindungen mit den Magnesiumverbindungen kann prinzipiell auch unter Beimischung von Lösungsmitteln erfolgen, wobei diese Lösungsmittel den Aufwand bei der Herstellung erhöhen, weil man diese nach der Umsetzung wieder entfernen muß. Wichtiger ist jedoch, daß bedingt durch Fremdlösungsmittel die Materialeigenschaften der Magnesiumhydroxide hinsichtlich der Reinheit und Einheitlichkeit der Porenverteilung und Kristallinität ungünstig beeinflußt werden. Weiterhin werden die Magnesiumverbindungen oft nach Umsetzung in anderen Lösungsmitteln und Aufarbeitung fest und unlöslich, so daß sie erst wieder in einem unpolaren Lösungsmittel aufgenommen werden müssen, bevor die Lösung einer Hydrolyse unterworfen werden kann. Die Umsetzung in anderen Lösungsmittel führt zu amorphen Magnesiumhydroxiden.

Erfindungsgemäß zur Herstellung von hochreinem Magnesiumhydroxid geeignete Magnesiumalkoxy-Verbindungen sind beispielsweise die Versuchsprodukte (VP.) Bis(ethylglykolato)-Magnesium (VP. 1), Bis(n-butylglykolato)-Magnesium (VP. 2), Magnesium-bis(N,N-dimethylamino-1-propanolat) (VP. 3), Magnesium-bis(N,N-dimethylaminoethanolat) (VP. 4) oder Magnesium-bis(2-ethylaminoethanolat) (VP.5) oder Magnesium-bis(1-Methoxy-2-propanolat) (VP.6).

Das erfindungsgemäß hergestellte Magnesiumhydroxid besitzt eine hohe Reinheit. Insbesondere sind die für die Anwendung in der Katalyse besonders nachteiligen Alkaliund Erdalkalimetallgehalte sehr gering. In Tabelle 1 sind die Ergebnisse der Spurenelementbestimmung mittels ICP dargelegt. Zum Vergleich sind käuflich erworbenes Magnesiumhydroxid "reinst" und Magenesiumoxid in p. a. Qualität herangezogen.

**Tabelle 1:**

| Spurenelementbestimmung mittels ICP | | | | | | | | |
|---|---|---|---|---|---|---|---|---|
| **Verbindung** | **Fe** [ppm] | **Si** [ppm] | **Ti** [ppm] | **Mn** [ppm] | **Zn** [ppm] | **Ga** [ppm] | **Na** [ppm] | **Ca** [ppm] |
| Magnesium | 286 | 46 | 3 | 8 | 23 | <5 | 23 | 9 |
| Mg-alkoxid | <2 | <2 | <1 | <1 | <2 | <5 | <2 | <1 |
| Mg(OH)₂ a | 12 | <2 | 3 | 3 | <2 | <5 | 14 | 6 |
| Mg(OH)₂ b | 52 | 699 | 1 | 7 | 2 | <5 | 58 | 2290 |
| MgO c | 9 | 51 | 1 | 6 | 2 | 4 | 1375 | 168 |
| MgO d | 15 | 7 | 3 | 3 | <2 | <5 | 16 | 10 |
| Legende: a: erfindungsgemäß hergest. Magnesiumhydroxid | | | | | | | | |
| b: Vergleichssubstanz Qualität "reinst" (von E. Merck AG, Darmstadt) | | | | | | | | |
| c: Vergleichssubstanz Qualität "p.a." (von Riedel de Haën AG, Hannover) | | | | | | | | |
| Mg-alkoxid: Bis(ethylglykolato)-magnesium | | | | | | | | |
| d: erfindungsgemäß hergest. MgO aus Mg(OH)₂ | | | | | | | | |

Aus dem Vergleich der Daten für die Verunreinigungen des Magnesiummetalls und der daraus hergestellten Magnesiumalkoxid-Verbindung wird ersichtlich, daß eine sehr effektive Abtrennung von Verunreinigungen z.B. von anderen Metallen gelingt. Als "hochrein" im Sinne der Erfindung werden Magnesiumoxide bzw. Magnesiumhydroxide mit folgenden Grenzwerten definiert:

**Tabelle 2:**

| Grenzwerte | | | | | | | | |
|---|---|---|---|---|---|---|---|---|
| **Verbindung** | **Fe** [ppm] | **Si** [ppm] | **Ti** [ppm] | **Mn** [ppm] | **Zn** [ppm] | **Ga** [ppm] | **Na** [ppm] | **Ca** [ppm] |
| | <50 | <50 | <10 | <10 | <10 | <10 | <50 | <50 |

insbesondere mit den Grenzwerten aus Tabelle 3, wobei der niedrige Grenzwert für die Alkali- und Erdalkaliionen, insbesondere Na und Ca ausschlaggebend ist, weil er diese Materialien für viele katalytische Anwendungen, die gegenüber Alkali- und Erdalkalifremdionen sensibel sind, prädestiniert:

**Tabelle 3:**

| Grenzwerte | | | | | | | | |
|---|---|---|---|---|---|---|---|---|
| **Verbindung** | **Fe** [ppm] | **Si** [ppm] | **Ti** [ppm] | **Mn** [ppm] | **Zn** [ppm] | **Ga** [ppm] | **Na** [ppm] | **Ca** [ppm] |
| | <20 | <20 | <5 | <5 | <5 | <5 | <20 | <20 |

Eine weitere Erhöhung der Reinheit gegenüber den Werten aus Tabelle 1 kann durch Verwendung von bidestilliertem Wasser und Gefäßen aus inertem Material erzielt werden. Die Hydrolyse durch "lediglich" deionisiertes Wasser erhöht den Fe-, Mn-, Ti-, Na- und Ca- Gehalt in dem Mg(OH)₂ (Verbindung a) geringfügig im Vergleich zur eingesetzten Magnesiumalkoxid-Verbindung (Mg-alkoxid).

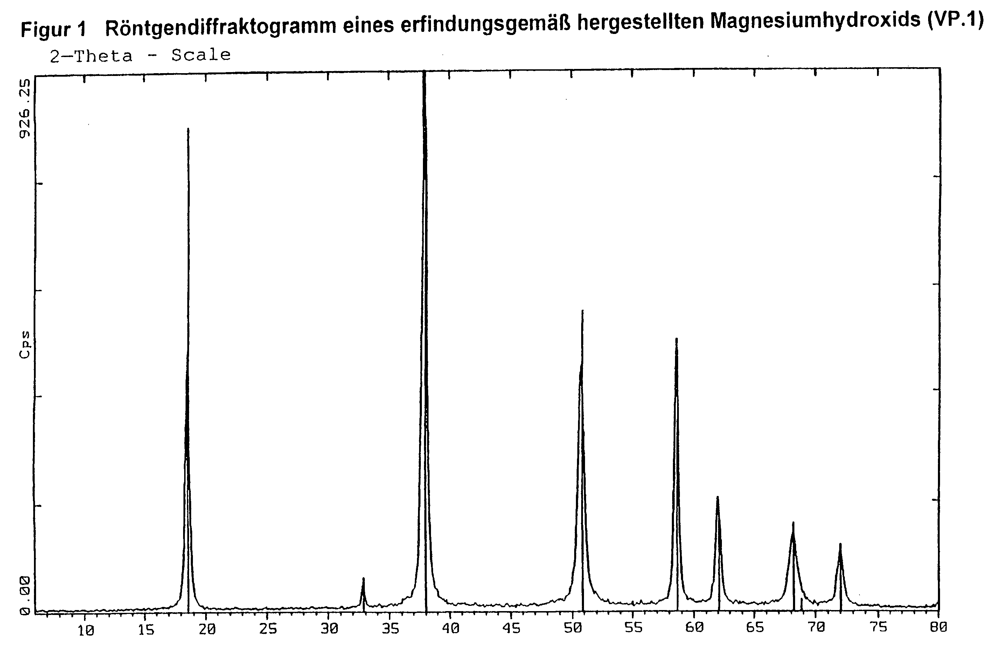

In Figur 1 ist das Röntgendiffraktogramm eines erfindungsgemäß dargestellten Magnesiumhydroxids dargestellt. Das Strichspektrum in Figur 1 gibt zum Vergleich das Röntgendiffraktogramm aus der JCPDS-Kartei (Eintrag Nr. 7-0239 Mg(OH)₂, Brucit, syn) wieder.

Aus den erfindungsgemäß hergestellten Verbindungen lassen sich durch Kalzinieren die Metalloxide herstellen. Zum Kalzinieren wurden die erfindungsgemäßen Verbindungen in einen Ofen bei Temperaturen zwischen 550 °C und 1500 °C über einen Zeitraum von 3 h bis 24 h verbracht. Das so hergestellte Metalloxid weist die gleiche hohe Reinheit wie das erfindungsgemäße Metallhydroxid auf.

In Tabelle 4 sind die Werte für einige physikalische Daten des erfindungsgemäß hergestellten Magnesiumhydroxids aufgeführt.

**Tabelle 4:**

| Physikalische Daten des Versuchsproduktes VP.1 | | | | | |
|---|---|---|---|---|---|
| **H**_{**2**}**0:Alkoholat** [g/g] | **Oberfläche** [m²/g] | **Porenvolumen** [ml/g] | **Porenradius** [Å] | **Hydrolysewasser** [pH] | **Hydrolysewasser** T [°C] |
| 1,15:1 | 142 | 0,71 ¹⁾ | 94 | 7 | 90 |
| 2 : 1 | 123 | 0,62 ¹⁾ | 68 | 7 | 90 |
| 4 : 1 | 142 | 0,62 ²⁾ | 88 | 7 | 90 |
| 4 : 1 | 97 | 0,76 ¹⁾ | 283 | 1 | 90 |
| 4 : 1 | 131 | 0,40 ¹⁾ | 78 | 4 | 90 |
| Legende: ¹⁾: Messung mittels Quecksilberporosimetrie (Porosimeter Autopore II 9220, Micromeritics) ²⁾: Messung mittels Stickstoffporosimetrie (ASAP 2010, Mikromeritics) | | | | | |

Geht man davon aus, daß der Porenradius mit den Kristallitgrößen korreliert, so wird ersichtlich, daß in Abhängigkeit vom pH-Wert des Hydrolysewassers, der z.B. durch Zugabe von Ammoniak zum Hydrolysewasser eingestellt werden kann, der gewünschte Porenradius und damit auch die gewünschte Kristallitgröße des erfindungsgemäß hergestelltem Magnesiumhydroxids erhalten werden kann.

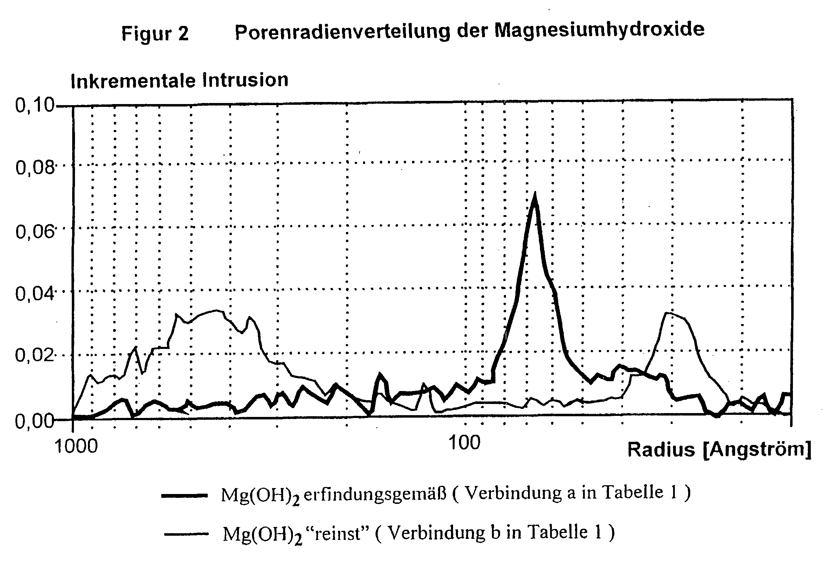

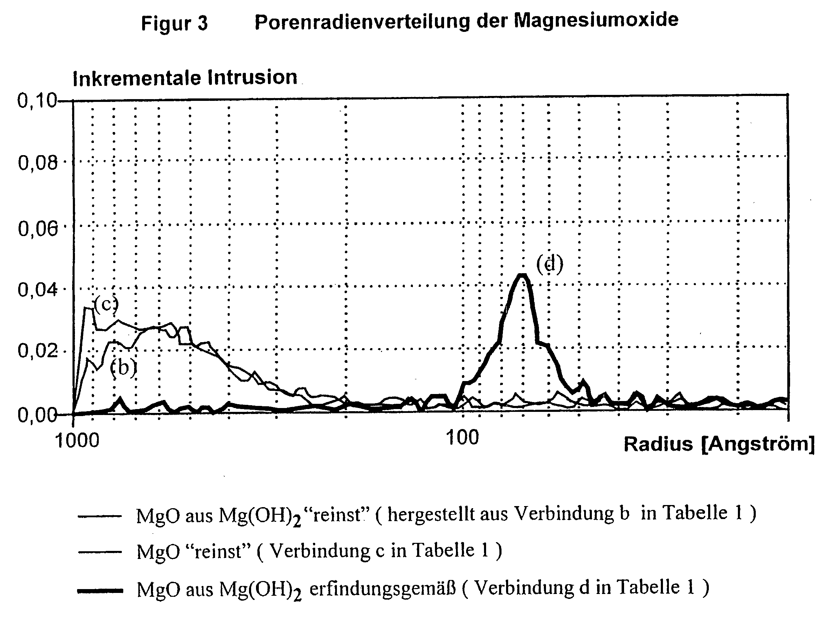

Die Verteilung der Porenradien der erfindungsgemmäß hergestellten Magnesiumhydroxid- und Magnesiumoxidproben ist wesentlich enger und einheitlicher (monomodale Verteilungen) als die herkömmlicher kommerziell erhältlicher Magnesiumhydroxide/oxide. Die Porenradienverteilung beider Produkte, Magnesiumhydroxid und Magnesiumoxid, ist in den Figuren 2 und 3 vergleichend dargestellt. Enge wohldefinierte Porenradien sind für katalytische Anwendungen von Bedeutung.

Zur Analyse der erfindungsgemäß hergestellten Verbindungen wurden die Verunreinigungen durch Spurenelemente mittels induktiv gekoppelter Plasmaspektroskopie (ICP) bestimmt. Die Bestimmung der kristallinen Phasen erfolgte durch Pulverdiffraktometrie. Oberflächen wurden mittels BET (3-Punkt Methode) ermittelt, Porenvolumina und Porenradien zusätzlich mittels Quecksilberporosimetrie sowie mittels Stickstoffporosimetrie. Zum Kalzinieren wurden die erfindungsgemäßen Verbindungen in einem Muffelofen Temperaturen zwischen 550 °C und 1500 °C ausgesetzt. Zur Hydrolyse wurde deionisiertes Wasser verwendet.

### Beispiel 1

### Umsetzung mit Ethylglycol (stöchiometrische Menge an CH₃-CH₂-O-CH₂-CH₂-OH) ammoniakalische Hydrolyse (VP. 1)

In einen 1000 ml Dreihalskolben wurden 20 g Magnesium-Granulat vorgelegt. Dazu wurden 48,4 g Ethylglycol gegeben. Die Mischung wurde erhitzt. Bei ca. 125 °C begann die Umsetzung des Metalls mit dem Ethylglycol, erkennbar an der Entwicklung von Wasserstoff und einem Temperaturanstieg auf ca. 140 °C. Man tropfte nun mittels eines Tropftrichters weitere 100 g Ethylglycol über einen Zeitraum von 30 min. zu. Das flüssige Reaktionsgemisch wurde filtriert. 100 g des Filtrats wurden in drei aliquoten Teilen in einer Vorlage (H₂O : Alkoholat = 4 : 1) bestehend aus 400 g deionisiertem Wasser (enthielt 0,2 Gew.%-. Ammoniak) hydrolysiert (T = 90 °C). Es entstand sofort ein weißer Niederschlag. Der freiwerdende Alkohol wurde abdestilliert. Der so erhaltene Slurry (Aufschlämmung) wurde sprühgetrocknet. Die Ausbeute betrug entsprechend 98 % der Theorie.

### Beispiel 1a

### Umsetzung mit Ethylglycol (VP. 1)

In einen 1000 ml Dreihalskolben wurden 20 g Magnesium-Granulat vorgelegt. Dazu wurden 50 g Ethylglycol gegeben. Die Mischung wurde erhitzt. Bei ca. 125 °C begann die Umsetzung des Metalls mit dem Ethylglycol, erkennbar an der Entwicklung von Wasserstoff und einem Temperaturanstieg auf ca. 140 °C. Man tropfte nun mittels eines Tropftrichters weitere 236 g Ethylglycol über einen Zeitraum von 50 min. zu. Das Reaktionsgemisch wurde filtriert. 100 g des Filtrats wurden in drei aliquoten Teilen in einer Vorlage (H₂O : Alkoholat = 4 : 1) bestehend aus 400 g deionisiertem Wasser (enthält 0,2 Gew.%. Ammoniak) hydrolysiert (T = 90 °C). Es entstand sofort ein weißer Niederschlag. Der freiwerdende Alkohol wurde abdestilliert. Der so erhaltene Slurry wurde sprühgetrocknet. Die Ausbeute beträgt entsprechend 98 % der Theorie.

### Beispiel 2

### Umsetzung mit Ethylglycol und Hexanol, ammoniakalische Hydrolyse (VP. 1)

In einen 1000 ml Dreihalskolben wurden 20 g Magnesium-Granulate vorgelegt. Dazu gibt man 20 g eines Hexanol/Ethylglycol (50 : 50 Gew.%). Die Mischung wurde erhitzt. Bei ca. 125 °C begann die Umsetzung des Metalls mit dem Ethylglycol/Hexanol-Gemisch, erkennbar an der Entwicklung von Wasserstoff und einem Temperaturanstieg auf ca. 140 °C. Man tropfte nun mittels eines Tropftrichters 266 g des Ethylglycols/Hexanols über einen Zeitraum von 50 min. zu. Das Reaktionsgemisch war selbst bei Raumtemperatur noch flüssig und wird filtriert. 100 g des Filtrats wurden in drei aliquoten Teilen in einer Vorlage (H₂O : Alkoholat = 4 : 1) bestehend aus 400 g deionisiertem Wasser (enthält 0,2 Gew.%-. Ammoniak) hydrolysiert (T = 90 °C). Es entstand sofort ein weißer Niederschlag. Der freiwerdende Alkohol wurde abdestilliert. Der so erhaltene Slurry wurde sprühgetrocknet. Die Ausbeute betrug entsprechend 98 % der Theorie.

### Beispiel 3

### Umsetzung mit n-Butylglycol (CH₃-CH₂-CH₂-CH₂-O-CH₂-CH₂-OH) ammoniakalische Hydrolyse (VP. 2)

In einen 1000 ml Dreihalskolben wurden 15 g Magnesium-Granulat vorgelegt. Dazu wurden 15 g n-Butylglykol gegeben. Die Mischung wurde erhitzt. Bei ca. 150 °C begann die Umsetzung des Metalls mit dem n-Butylglykol, erkennbar an der Entwicklung von Wasserstoff. Die Reaktionstemperatur pegelte sich bei ca. 180 °C ein. Man tropfte nun mittels eines Tropftrichters weitere 252 g n-Butylglykol über einen Zeitraum von 90 min. zu. Das Reaktionsgemisch wurde filtriert. 100 g des Filtrats wurden in drei aliquoten Teilen in einer Vorlage (H₂O : Alkoholat = 4 : 1) bestehend aus 400 g deionisiertem Wasser (enthält 0,2 Gew.%- Ammoniak) hydrolysiert (T = 90 °C). Es entstand sofort ein weißer Niederschlag. Der freiwerdende Alkohol wurde abdestilliert. Der so erhaltene Slurry wurde sprühgetrocknet. Die Ausbeute betrug entsprechend 98 % der Theorie.

### Beispiel 3a

### Umsetzung mit n-Butylglycol, Hydrolyse ohne Ammoniak (stöchiometrische Menge an n-Butylglykol) (VP. 2)

In einen 1000 ml Dreihalskolben wurden 15 g Magnesium-Granulate vorgelegt. Dazu wurden 25,7 g n-Butylglykol gegeben. Die Mischung wurde erhitzt. Bei ca. 155 - 160 °C begann die Umsetzung des Metalls mit dem n-Butylglykol, erkennbar an der Entwicklung von Wasserstoff. Man tropfte nun mittels eines Tropftrichters weitere 120 g n-Butylglykol über einen Zeitraum von 90 min. zu. Das flüssige Reaktionsgemisch wurde filtriert. 100 g des Filtrats wurde in drei aliquoten Teilen in einer Vorlage (H₂O : Alkoholat = 4 : 1) bestehend aus 400 g deionisiertem Wasser hydrolysiert (T = 90 °C). Es entstand sofort ein weißer Niederschlag. Der freiwerdende Alkohol wurde abdestilliert. Der so erhaltene Slurry wurde sprühgetrocknet. Die Ausbeute betrug entsprechend 98 % der Theorie.

### Beispiel 3b

### Umsetzung mit n-Butylglycol, Hydrolyse ohne Ammoniak (VP. 2)

In einen 1000 ml Dreihalskolben wurden 15 g Magnesium-Granulate vorgelegt. Dazu wurden 15 g n-Butylglykol gegeben. Die Mischung wurde erhitzt. Bei ca. 155-160 °C begann die Umsetzung der Metalle mit dem n-Butylglykol, erkennbar an der Entwicklung von Wasserstoff. Man tropfte nun mittels eines Tropftrichters weitere 252 g n-Butylglykol über einen Zeitraum von 90 min. zu. Das Reaktionsgemisch wurde filtriert. 100 g des Filtrats wurden in drei.aliquoten Teilen in einer Vorlage (H₂O : Alkoholat = 4 : 1) bestehend aus 400 g deionisiertem Wasser hydrolysiert (T = 90 °C). Es entstand sofort ein weißer Niederschlag. Der überstehende Alkohol wurde abdekantiert. Der freiwerdende Alkohol wurde abdestilliert. Der so erhaltene Slurry wurde sprühgetrocknet. Die Ausbeute betrug entsprechend 98 % der Theorie.

### Beispiel 4

### Umsetzung mit 3-Dimethylamino-1-propanol (VP. 3)

In einen 1000 ml Dreihalskolben wurden 15 g Magnesium-Granulate vorgelegt. Dazu wurden 50 g 3-Dimethylamino-1-propanol gegeben. Die Mischung wurde erhitzt. Bei ca. 150 - 160 °C begann die Umsetzung der Metalle mit 3-Dimethylamino-1-propanol, erkennbar an der Entwicklung von Wasserstoff. Man tropfte nun mittels eines Tropftrichters weitere 197 g 3-Dimethylamino-1-propanol über einen Zeitraum von 6 h zu. Das Reaktionsgemisch wurde filtriert. 100 g des Filtrats werden in drei aliquoten Teilen in einer Vorlage (H₂O : Alkoholat 4 : 1) bestehend aus 400 g deionisiertem Wasser hydrolysiert (T = 90 °C). Es entstand sofort ein weißer Niederschlag. Der freiwerdende Alkohol wurde abdestilliert. Der so erhaltene Slurry wurde sprühgetrocknet. Die Ausbeute betrug entsprechend 95% der Theorie.

### Beispiel 5

### Umsetzung mit 2-(Dimethylamino)ethanol (VP. 4)

In einen 1000 ml Dreihalskolben wurden 15 g Magnesium-Granulate vorgelegt. Dazu wurden 50 g 2-Dimethylaminoethanol gegeben. Die Mischung wurde erhitzt. Bei ca. 135 - 145 °C beginnt die Umsetzung der Metalle mit 2-Dimethylaminoethanol, erkennbar an der Entwicklung von Wasserstoff. Man tropfte nun mittels eines Tropftrichters weitere 321 g 2-Dimethylaminoethanol über einen Zeitraum von 5 h zu. Das Reaktionsgemisch wurde filtriert. 100 g des Filtrats wurden in drei aliquoten Teilen in einer Vorlage (H₂O : Alkoholat 4 : 1) bestehend aus 400 g deionisiertem Wasser hydrolysiert (T = 90 °C). Es entstand sofort ein weißer Niederschlag. Der freiwerdende Alkohol wurde abdestilliert. Der so erhaltene Slurry wurde sprühgetrocknet. Die Ausbeute betrug entsprechend 96 % der Theorie.

### Beispiel 6

### Umsetzung mit 2-Ethylamino-ethanol (VP. 5)

In einen 1000 ml Dreihalskolben wurden 15 g Magnesium-Granulate vorgelegt. Dazu gab man 50 g 2-Ethylamino-ethanol. Die Mischung wurde erhitzt. Bei ca. 150 - 160 °C beginnt die Umsetzung der Metalle mit 2-Ethylamino-ethanol, erkennbar an der Entwicklung von Wasserstoff. Man tropfte nun mittels eines Tropftrichters weitere 123 g 2-Ethylaminoethanol über einen Zeitraum von 4 h zu. Das Reaktionsgemisch wurde filtriert. 100 g des Filtrats wurden in drei aliquoten Teilen in einer Vorlage (H₂O : Alkoholat = 4 : 1) bestehend aus 400 g deionisiertem Wasser hydrolysiert (T = 90 °C). Es entstand sofort ein weißer Niederschlag. Der freiwerdende Alkohol wird abdestilliert. Der so erhaltene Slurry wurde sprühgetrocknet. Die Ausbeute betrug entsprechend 94 % der Theorie.

### Beispiel 7

### Umsetzung mit 1-(Methoxy)propan-2-ol (VP. 6)

In einen 500 ml Dreihalskolben wurden 10 g Magnesium-Granulate vorgelegt. Dazu gab man 15 g 1-Methoxy-2-propanol. Die Mischung wurde erhitzt. Bei ca. 120 °C begann die Umsetzung des Metalls mit 1-Methoxy-2-propanol, erkennbar an der Entwicklung von Wasserstoff. Man tropfte nun mittels eines Tropftrichters weitere 59,1 g 1-Methoxy-2-propanol über einen Zeitraum von 5 h zu. Das Reaktionsgemisch wurde filtriert. 50 g des Filtrats wurden in drei aliquoten Teilen in einer Vorlage (H₂O : Alkoholat = 4 : 1) bestehend aus 200 g deionisiertem Wasser hydrolysiert (T = 90 °C). Es entstand sofort ein weißer Niederschlag. Der freiwerdende Alkohol wird abdestilliert. Der so erhaltene Slurry wurde sprühgetrocknet. Die Ausbeute betrug entsprechend 80 % der Theorie.

## Patentansprüche

1. Verfahren zur Herstellung von hochreinem Magnesiumhydroxid, **dadurch gekennzeichnet, daß** man Magnesiummetall und/oder reaktive Magnesiumverbindungen
mit einer oder mehreren Hydroxy-Verbindungen des Typs
**R**^{**1**}**-A-R-OH** (I) ,
oder
neben den Hydroxy-Verbindungen des Typs R¹-A-R-OH (I) zusammen mit bis zu 50 Gew.%, bezogen auf die Gesamtheit aller eingesetzten Edukte oder Lösungsmittel, eines oder mehrerer Alkohole des Typs
**R**^{**2**}**-OH** (II) ,
wobei
(a) A für ein Element aus der Hauptgruppe 6 (Sauerstoff-Gruppe) oder Hauptgruppe 5 (Stickstoff-Gruppe) des Periodensystems steht, wobei wenn A für ein Element der Hauptgruppe 5 steht, A weitere Substituenten R¹ oder Wasserstoff trägt, die untereinander verschieden sein können, und
(b) R, R¹ und R² für einen verzweigten oder unverzweigten, cyclischen oder acyclischen, gesättigten, ungesättigten oder aromatischen Kohlenwasserstoffrest mit 1 - 10 Kohlenstoffatomen steht, wobei R, R¹ und R² voneinander verschieden sein können und R zweifach substituiert ist,
zu flüssigen / gelösten Magnesiumalkoxiden umsetzt und nach Abtrennung schwerer löslicher Verunreinigungen hydrolysiert.

2. Verfahren gemäß Anspruch 1, **dadurch gekennzeichnet, daß** man Magnesiummetall einsetzt.

3. Verfahren gemäß einem der vorhergehen Ansprüche, wobei R und R¹ für einen verzweigten oder unverzweigten, gesättigten Kohlenwasserstoffrest mit 1 bis 5 Kohlenstoffatomen steht, wobei R und R¹ voneinander verschieden sein können und R zweifach substituiert ist.

4. Verfahren gemäß einem der vorhergehenden Ansprüche, **dadurch gekennzeichnet, daß** R² für einen unverzweigten, acyclischen und gesättigten Kohlenwasserstoffrest mit 4 bis 8 Kohlenstoffatomen steht.

5. Verfahren gemäß einem der vorhergehenden Ansprüche, **dadurch gekennzeichnet, daß** man einen 1 bis 5-fachen Überschuß der Hydroxy-Verbindungen einsetzt, bezogen auf das stöchiometrische Verhältnis der Hydroxy-Gruppen zu den Mg-Valenzen.

6. Verfahren gemäß einem der vorhergehenden Ansprüche, **dadurch gekennzeichnet, daß** man ohne Zusatz von Lösungsmitteln im wesentlichen ausschließlich mit/in den Hydroxy-Verbindungen der Formel R¹-A-R-OH (I) oder den Hydroxy-Verbindungen der Formel R¹-A-R-OH (I) und R²-OH (II) zu den Magnesiumhydroxiden umsetzt.

7. Verfahren gemäß einem der vorhergehenden Ansprüche, **dadurch gekennzeichnet, daß** man die Hydroxy-Verbindungen der Formel R²-OH (II) erst nach Umsetzung des Metalls / der Metallverbindung mit den Hydroxy-Verbindungen der Formel R¹-A-R-OH (I) zugibt.

8. Verfahren zur Herstellung von Magnesiumoxiden, **dadurch gekennzeichnet, daß** man die Magnesiumhydroxide gemäß einem der vorhergehenden Ansprüche kalziniert.

9. Verwendung des gemäß einem der Ansprüche 1 bis 8 hergestellten Magnesiumhydroxids oder Magnesiumoxids als Katalysator und/oder Katalysatorträgermaterial für katalytische Prozesse bzw. zur Herstellung derselben.

10. Verwendung des gemäß einem der Ansprüche 1 bis 8 hergestellten Magnesiumhydroxids oder Magnesiumoxids als Ausgangsmaterial zur Herstellung von keramischen Werkstoffen oder keramischen Formkörpern.

## Claims

1. A process for producing high-purity magnesium hydroxide
**characterised in that** magnesium metal and/or reactive magnesium compounds are reacted with one or more hydroxy compound(s) of the type
**R**^{**1**}**-A-R-OH** (I),
or with the hydroxy compounds of the type R¹-A-R-OH (I) together with up to 50 percent by weight of one or more alcohol(s) of the type
**R**^{**2**}**-OH,** (II) ,
based on the total of all educts or solvents used, wherein
(a) **A** represents an element of main group 6 (oxygen group) or main group 5 (nitrogen group) of the periodic system, wherein if A represents an element of main group 5, **A** bears additional substituents R¹ or hydrogen which may be different from each other; and
(b) R, R¹, and R² represent a branched or unbranched, cyclic or acyclic, saturated, unsaturated, or aromatic hydrocarbon residue having 1 to 10 carbon atoms, wherein R, R¹, and R² may be different from each other and R is twofold substituted,
to liquid/dissolved magnesium alkoxides, and that less soluble impurities are separated and hydrolysis is performed.

2. A process according to claim 1,
**characterised in that** magnesium metal is used.

3. A process according to any one of the preceeding claims, wherein R and R¹ represent a branched or unbranched saturated hydrocarbon residue having 1 to 5 carbon atoms, wherein R and R¹ may be different from each other and R is twofold substituted.

4. A process according to any one of the preceeding claims,
**characterised in that** R² represents an unbranched, acyclic and saturated hydrocarbon residue having 4 to 8 carbon atoms.

5. A process according to any one of the preceeding claims,
**characterised in that** the hydroxy compounds are employed in a one- to fivefold excess, based on the stoichiometric ratio of the hydroxy groups to the Mg valences.

6. A process according to any one of the preceeding claims,
**characterised in that** the reaction is carried out without addition of solvents essentially exclusively with/in the hydroxy compounds of formula R¹-A-R-OH (I) or the hydroxy compounds of formula R¹-A-R-OH (I) and R²-OH (II) to form the magnesium hydroxides.

7. A process according to any one of the preceeding claims,
**characterised in that** the hydroxy compounds of formula R²-OH (II) are added only after the reaction of the metal / metal compound with the hydroxy compounds of formula R¹-A-R-OH (I).

8. A process for producing magnesium oxides
**characterised in that** the magnesium hydroxides according to any one of the preceeding claims are calcined.

9. Use of the magnesium hydroxide or magnesium oxide produced according to any one of claims 1 to 8 as catalyst and/or catalyst support for catalytic processes, or for the manufacture thereof.

10. Use of the magnesium hydroxide or magnesium oxide produced according to any one of claims 1 to 8 as starting material for the production of ceramic materials or ceramic die-formed parts.

## Revendications

1. Procédé pour la préparation d'hydroxyde de magnésium de haute pureté, **caractérisé en ce qu'**on transforme du magnésium métallique et/ou des composés réactifs du magnésium avec un ou plusieurs composés hydroxy du type
R¹-A-R-OH (I),
ou,
en plus des composés hydroxy du type R¹-A-R-OH (I), conjointement avec jusqu'à 50% en poids, par rapport à la totalité de tous les produits de départ ou solvants utilisés, d'un ou de plusieurs alcools du type
R²-OH (II),
où
(a) A représente un élément du 6ème groupe principal (groupe de l'oxygène) ou du 5ème groupe principal (groupe de l'azote) du système périodique, A portant d'autres substituants R¹ ou hydrogène, qui peuvent être différents les uns des autres lorsque A représente un élément du 5ème groupe principal, et
(b) R, R¹ et R² représentent un radical hydrocarboné ramifié ou non ramifié, cyclique ou acyclique, saturé, insaturé ou aromatique comprenant 1 à 10 atomes de carbone, R, R¹ et R² pouvant être différents les uns des autres et R étant disubstitué,
en alcoxydes de magnésium liquides/dissous et on hydrolyse après la séparation des impuretés difficilement solubles.

2. Procédé selon la revendication 1, **caractérisé en ce qu'**on utilise du magnésium métallique.

3. Procédé selon l'une quelconque des revendications précédentes, R et R¹ représentant un radical hydrocarboné ramifié ou non ramifié, saturé comprenant 1 à 5 atomes de carbone, R et R¹ pouvant être différents l'un de l'autre et R étant disubstitué.

4. Procédé selon l'une quelconque des revendications précédentes, **caractérisé en ce que** R² représente un radical hydrocarboné non ramifié, acyclique et saturé comprenant 4 à 8 atomes de carbone.

5. Procédé selon l'une quelconque des revendications précédentes, **caractérisé en ce qu'**on utilise un excès d'un facteur 1 à 5 de composés hydroxy par rapport au rapport stoechiométrique des groupements hydroxy aux valences de Mg.

6. Procédé selon l'une quelconque des revendications précédentes, **caractérisé en ce qu'**on transforme sans addition de solvants, de manière essentiellement exclusive avec/dans les composés hydroxy de formule R¹-A-R-OH (I) ou les composés hydroxy de formule R¹-A-R-OH (I) et R²-OH (II) en les hydroxydes de magnésium.

7. Procédé selon l'une quelconque des revendications précédentes, **caractérisé en ce qu'**on n'ajoute les composés hydroxy de formule R²-OH (II) qu'après la transformation du métal/du composé métallique avec les composés de formule R¹-A-R-OH (I).

8. Procédé pour la préparation d'oxydes de magnésium, **caractérisé en ce qu'**on calcine les hydroxydes de magnésium selon l'une quelconque des revendications précédentes.

9. Utilisation de l'hydroxyde de magnésium ou de l'oxyde de magnésium préparé selon l'une quelconque des revendications 1 à 8 comme catalyseur et/ou matériau support de catalyseur pour des processus catalytiques ou, selon le cas, pour la préparation de ceux-ci.

10. Utilisation de l'hydroxyde de magnésium ou de l'oxyde de magnésium préparé selon l'une quelconque des revendications 1 à 8 comme matériau de départ pour la fabrication de matériaux céramiques ou de corps façonnés céramiques.
